# EUROPEAN PATENT SPECIFICATION

(11) **EP 1 263 742 B1**
(45) Date of publication and mention of the grant of the patent: **24.08.2005**
(21) Application number: 01906529.1
(22) Date of filing: 07.02.2001
(51) Int. Cl.: C07D 263/22, A61K 31/42, A61P 31/00

(54) **OXAZOLIDINONE THIOAMIDES WITH PIPERAZINE AMIDE SUBSTITUENTS**
OXAZOLIDINON-THIOAMIDE MIT PIPERAZINAMID-SUBSTITUENTEN
THIOAMIDES D'OXAZOLIDINONE A SUBSTITUANTS AMIDES DE PIPERAZINE

(30) Priority: 10.02.2000 US 181640 P
(43) Date of publication of application: 11.12.2002
(73) Proprietor: Pharmacia & Upjohn Company LLC, Kalamazoo, MI 49001 (US)
(72) Inventor: HESTER, Jackson, B., Jr., Galesburg, MI 49053 (US)
(74) Representative: Perry, Robert Edward
(86) International application number: PCT/US2001/000682
(87) International publication number: WO 2001/058885

(56) References cited:
- EP-A- 0 074 268
- WO-A-00/27830
- WO-A-00/73301
- WO-A-98/54161
- WO-A-99/12914
- PATENT ABSTRACTS OF JAPAN vol. 2000, no. 02, 29 February 2000 (2000-02-29) & JP 11 322729 A (HOKURIKU SEIYAKU CO LTD), 24 November 1999 (1999-11-24)

## Description

### FIELD OF THE INVENTION

The present invention relates to novel oxazolidinone thioamides which have activities against gram-positive and gram-negative bacteria.

### BACKGROUND OF THE INVENTION

The oxazolidinone antibacterial agents are a class of antimicrobials with potent activity against a number of human and veterinary pathogens, including gram-positive aerobic bacteria such as multiply-resistant staphylococci and streptococci, anaerobic organisms such as bacteroides and clostridia species, and acid-fast organisms such as *Mycobacterium tuberculosis* and *Mycobacterium avium.*

However, oxazolidinones generally do not demonstrate activity at a useful level, against aerobic gram-negative organisms. Thus, their use is limited to infectious states due to gram-positive bacteria.

WO98/54161 discloses oxazolidinone antibacterial agents having a thiocarbonyl functionality.

WO93/23384 discloses oxazolidinones containing a substituted diamine moiety and their use as antimicrobials.

WO95/07271 discloses substituted oxazine and thiazine oxazolidinones and their use as antimicrobial.

WO99/12914 discloses antimicrobial thiourea derivatives.

Patent Abstracts of Japan vol. 2000, no. 02, 29 February 2000, and JP 11 322729 A, disclose oxazolidinones and their use as antimicrobial.

### SUMMARY OF THE INVENTION

Novel compounds according to the present invention are of formula I 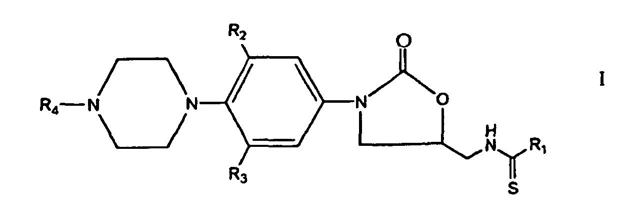 or a pharmaceutically acceptable salt thereof, wherein
R₁ is H, C₂₋₄ alkenyl, -(CH₂)₀₋₂-C₃₋₆ cycloalkyl, or C₁₋₄ alkyl optionally substituted with 1-3 F, 1-2 Cl or CN;
R₂ and R₃ are independently H, F, Cl or C₁₋₂ alkyl; and
R₄ is
(a) -CO-CHPh-O-C₁₋₄alkanoyl,
(b) -CO-CH₂-S(O)₀₋₂-CH₃,
(c) -CS-C₁₋₄ alkyl,
(d) -CO-CH₂-O-CO₂-(CH₂)₂-OCH₃,
(e) -CO-CH₂-O-CO-NH-C₁₋₃ alkyl,
(f) -CO-(CH₂)₀₋₁-CO-CH₃, or
(g) -CN.

According to another aspect of the invention, a pharmaceutical composition comprises a compound of formula I and a pharmaceutically acceptable carrier. According to a further aspect, a compound of formula I is used for the manufacture of a medicament, for treating a microbial infection in a mammal.

Compounds of the invention may be used to treat gram-positive and gram-negative organisms. In particular, the compounds of the invention increase the spectrum of activity of oxazolidinone antimicrobials to include gram-negative organisms such as *Haemophilus influenza* and *Moraxella catarrhalis*.

### DETAILED DESCRIPTION OF THE INVENTION

The following definitions are used, unless otherwise described.

The term alkyl, alkenyl, etc. refer to both straight and branched groups, but reference to an individual radical such as "propyl" embraces only the straight chain radical, a branched chain isomer such as "isopropyl" being specifically referred to.

The carbon atom content of various hydrocarbon-containing moieties is indicated by a prefix designating the minimum and maximum number of carbon atoms in the moiety, i.e., the prefix Cᵢ₋ⱼ indicates a moiety of the integer "i" to the integer "j" carbon atoms, inclusive. Thus, for example, C₁₋₇ alkyl refers to alkyl of one to seven carbon atoms, inclusive.

Mammal refers to human or animals.

The compounds of the present invention are generally named according to the IUPAC or CAS nomenclature system. Abbreviations which are well known to one of ordinary skill in the art may be used (e.g. "Ph" for phenyl, "Me" for methyl, "Et" for ethyl, "h" for hour or hours and "rt" for room temperature).

Specific and preferred values listed below for radicals, substituents, and ranges, are for illustration only; they do not exclude other defined values or other values within defined ranges for the radicals and substituents.

A specific value for R₁ is C₁₋₄ alkyl, e.g. ethyl.

A specific value for each of R₂ and R₃ is H or F; for example, R₂ is H and/or R₃ is F. Specific values for R₄ are C(=O)-CH(CH₂-phenyl)(OH), C(=O)-CH₂-SO₂-CH₃, C(=O)-CH(OH)(CH₂OH), C(=O)-C(=O)-CH₃, C(=O)-CH(OH)(CH₂-O-CH₃), C(O)-CH₂CH₂-OH, C(=O)-CH₂-O-CO₂-(CH₂)₂-OCH₃, C(=S)-CH₃, and CN.

Preferred compounds of the present invention are those of formula la

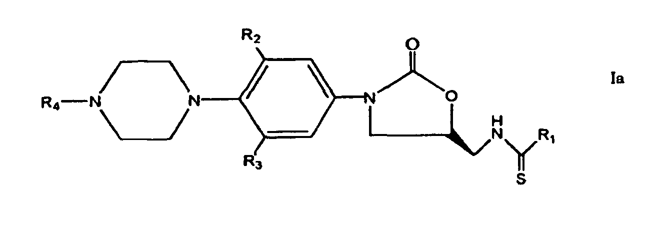

This absolute configuration is the (S)-configuration according to the Cahn-Ingold-Prelog nomenclature system. It will be appreciated by those skilled in the art that compounds of the present invention may have additional chiral centers and be isolated in optically active or racemic form. The present invention encompasses any racemic, optically-active, tautomeric, or stereoisomeric form, or mixture thereof, of a compound of the invention.

Preferred specific compounds of the invention are given in the Examples and claims 7 to 12.

Scheme I describes the preparation of compounds of the present invention. All of the starting materials are prepared by procedures described in these schemes or by procedures that would be well known to one of ordinary skill in organic chemistry. The variables used in Schemes I are as defined below or as in the claims. Optically pure material could be obtained either by one of a number of asymmetric syntheses or alternatively by resolution from a racemic mixture.

In step 1 of Scheme I, a suitably protected piperazine (II) is allowed to react with an activated carboxylic acid derivative to give compounds III. In this reaction activated carboxylic acid derivatives can include acyl halides and acid anhydrides or mixed anhydrides which are allowed to react with II in the presence of a tertiary amine base such as triethylamine or pyridine in solvents such as methylene chloride, tetrahydrofuran (THF) or excess pyridine. Temperatures in the range of about 0°C to about 24°C are generally suitable for this reaction. Alternatively coupling agents which are well known for amide forming reactions can be used with appropriate carboxylic acids in step 1. Reagents such as dicyclohexylcarbodiimide (DCC) and 1-(3-dimethylaminopropyl)-3-ethylcarbodiimide hydrochloride (EDC) can be used with activating agents such as 1-hydroxybenzotriazole (HOBT) or 4-(dimethylamino)pyridine (DMAP) in this reaction. Solvents such as THF or dimethylformamide (DMF) and temperatures in the range of 0°C to 24°C are suitable. Compounds where R₄ is cyano are prepared by allowing compounds II to react with cyanogen bromide in solvents such as methanol. Sodium acetate is a suitable base for this reaction which can be carried out at temperatures in the range of 0°C to 24°C (See Example 5). Protecting groups (P) are chosen for their compatibility with other functional groups on the molecule. Benzyloxycarbonyl (Cbz) and *tert*-butoxycarbonyl (Boc) are generally suitable protecting groups for these compounds; however, it is sometimes necessary to employ other protecting groups. Example 1 illustrates the use of the phthalimide protecting group. In this example, the sulfoxide is sensitive to the acidic conditions required for Boc group removal. The phthalimide can be removed under non-acidic conditions with hydrazine hydrate or methylamine.

In step 2 of Scheme 1, the protecting group (P) is removed to give the corresponding amines (IV). It is convenient to remove the Boc group with hydrogen chloride in dioxane at 0°C to 24°C; however, other deprotection strategies can be employed. Deprotection of Cbz groups can generally be accomplished by hydrogenation with a palladium catalyst.

In step 3 of Scheme 1, the amines (IV) are converted to compounds of formula I. Thioamides are prepared by allowing compounds IV to react with dithioesters and a tertiary amine base such as triethylamine. In this reaction it is often convenient to employ an excess of the tertiary amine base with an amine salt prepared by Boc deprotection in step 2 without first isolating the free base. Solvents such as THF, methylene chloride or preferably methanol and temperatures in the range of about 24°C to about 50°C can be used for this reaction. Other thiocarbonyl compounds of formula I can be prepared according to the procedures disclosed in PCT International Publication WO 98/54161.

If desired R₄ of compounds I or III can be modified to give additional compounds of formula L This is illustrated in Example 4 where the acetamide of **14** is allowed to react with Lawesson's reagent to give the thioamide **15,** a compound of formula I. In Example 1 it is shown in step **3** that the sulfide **3** can be oxidized to the sulfoxide **4** with sodium periodate in methanol-water. This intermediate **4** can subsequently be converted to a compound of formula L The reaction of sulfoxides such as **4** (Example 1) with sodium azide in polyphosphoric acid at temperatures in the range of 40°C to 70°C gives sulfoximine intermediates that can also be converted to the corresponding compounds of formula I (R₄ is -C(=O)-CH₂-S(=O)(=NR₈)CH₃,). Other sulfoximine analogs can be obtained as described in Case 6295. In Example 3 it is shown that the sulfide intermediate **8** can be oxidized to the sulfone **11** with osmium tetroxide and 4-methylmorpholine N-oxide in acetone-water. Intermediate **11** can subsequently be converted to compound **13,** a compound of formula I. In Example 6 it is shown that the alcohol **19** will react with methylisocyanate and a cuprous chloride catalyst in DMF at 24°C to give **20** which can be converted to **22,** a compound of formula I. And in Example 7 acylation of the alcohol **23** with 2-methoxyethyl chloroformate in pyridine gives **24,** a compound of formula I. In a similar manner, using chemistry known in the art, other R₄ substituents of compounds I or III of Scheme I can be modified to give additional compounds of formula I.

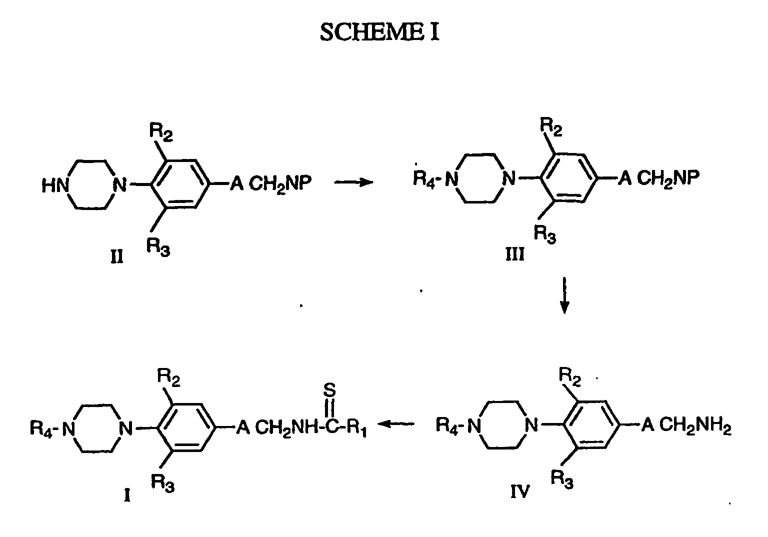

The pharmaceutical compositions of this invention may be prepared by combining the compounds of formula I of this invention with a solid or liquid pharmaceutically acceptable carrier and, optionally, with pharmaceutically acceptable adjuvants and excipients employing standard and conventional techniques. Solid form compositions include powders, tablets, dispersible granules, capsules, cachets and suppositories. A solid carrier can be at least one substance which may also function as a diluent, flavoring agent, solubilizer, lubricant, suspending agent, binder, tablet disintegrating agent, and encapsulating agent. Inert solid carriers include magnesium carbonate, magnesium stearate, talc, sugar, lactose, pectin, dextrin, starch, gelatin, cellulosic materials, low melting wax, cocoa butter, and the like. Liquid form compositions include solutions, suspensions and emulsions. For example, there may be provided solutions of the compounds of this invention dissolved in water and water-propylene glycol and water-polyethylene glycol systems, optionally containing suitable conventional coloring agents, flavoring agents, stabilizers and thickening agents.

Preferably, the pharmaceutical composition is provided employing conventional techniques in unit dosage form containing effective or appropriate amounts of the active component, that is, the compounds of formula I according to this invention.

The quantity of active component, that is the compound of formula I according to this invention, in the pharmaceutical composition and unit dosage form thereof may be varied or adjusted widely depending upon the particular application, the potency of the particular compound and the desired concentration. Generally, the quantity of active component will range between 0.5% to 90% by weight of the composition.

In therapeutic use for treating, or combating, bacterial infections in warm-blooded animals, the compounds or pharmaceutical compositions thereof will be administered orally, topically, transdermally, and/or parenterally at a dosage to obtain and maintain a concentration, that is, an amount, or blood-level of active component in the animal undergoing treatment which will be antibacterially effective. Generally, such antibacterially effective amount of dosage of active component will be in the range of about 0.1 to about 100, more preferably about 1.0 to about 50 mg/kg of body weight/day. It is to be understood that the dosages may vary depending upon the requirements of the patient, the severity of the bacterial infection being treated, and the particular compound being used. Also, it is to be understood that the initial dosage administered may be increased beyond the above upper level in order to rapidly achieve the desired blood-level or the initial dosage may be smaller than the optimum and the daily dosage may be progressively increased during the course of treatment depending on the particular situation. If desired, the daily dose may also be divided into multiple doses for administration, e.g., two to four times per day.

The compounds of formula I according to this invention are administered parenterally, i.e., by injection, for example, by intravenous injection or by other parenteral routes of administration. Pharmaceutical compositions for parenteral administration will generally contain a pharmaceutically acceptable amount of the compound according to formula I as a soluble salt (acid addition salt or base salt) dissolved in a pharmaceutically acceptable liquid carrier such as, for example, water-for-injection and a buffer to provide a suitably buffered isotonic solution, for example, having a pH of about 3.5-6. Suitable buffering agents include, for example, trisodium orthophosphate, sodium bicarbonate, sodium citrate, N-methylglucamine, L(+)-lysine and L(+)-arginine to name but a few representative buffering agents. The compounds according to formula I generally will be dissolved in the carrier in an amount sufficient to provide a pharmaceutically acceptable injectable concentration in the range of about 1 mg/ml to about 400 mg/ml of solution. The resulting liquid pharmaceutical composition will be administered so as to obtain the above-mentioned antibacterially effective amount of dosage. The compounds of formula I according to this invention are advantageously administered orally in solid and liquid dosage forms.

The oxazolidinone antibacterial agents of this invention have useful activity against a variety of organisms. The in vitro activity of compounds of this invention can be assessed by standard testing procedures such as the determination of minimum inhibitory concentration (MIC) by agar dilution as described in "Approved Standard. Methods for Dilution Antimicrobial Susceptibility Tests for Bacteria That Grow Aerobically", 3rd. ed., published 1993 by the National Committee for Clinical Laboratory Standards, Villanova, Pennsylvania, USA. The activity of compounds of this invention against *Staphylococcus aureus, Staphylococcus epidermidis, Streptococcus pneumoniae, Enterococcus faecalis, Moraxella catarrhalis* and H. *influenzae* is shown in Table 1.

**TABLE 1**

| Antibacterial Activity Minimum Inhibitory Concentration (µg/mL) | | | | | | | | |
|---|---|---|---|---|---|---|---|---|
| | SAUR 9213 MIC | SEPI 30593 MIC | EFAE 12712 MIC | SPNE 9912 MIC | HINF 30063 MIC | HINF 30063 MIC | EFAE 9217 MIC | MCAT 30607 MIC |
| EX 1 | 4 | 1 | 2 | .05 | 2 | 8 | 1 | 8 |
| EX 2 | 2 | 1 | 1 | <0.5 | 1 | >64 | 1 | 4 |
| EX 3 | 2 | 0.5 | 1 | 0.25 | 0.5 | 8 | 0.5 | 2 |
| EX 4 | 0.5 | 0.25 | 0.5 | 0.125 | 0.125 | 1 | 0.5 | 1 |
| EX 5 | 0.25 | 0.25 | 0.25 | 0.125 | 0.125 | 2 | 0.25 | 1 |
| EX 7 | 2 | 1 | 2 | 0.25 | 0.25 | 4 | 2 | 4 |
| EX 8 | 2 | 1 | 2 | 0.5 | 0.5 | 16 | 1 | 2 |
| EX 9 | 4 | 2 | 4 | 1 | 1 | 32 | 2 | 4 |
| EX 10 | 4 | 2 | 2 | 1 | 1 | 16 | 1 | 4 |
| EX 12 | 2 | 1 | 1 | 0.5 | 0.5 | 8 | 1 | 2 |
| EX 13 | 2 | 1 | 1 | 0.5 | 0.5 | 8 | 1 | 2 |
| EX 14 | 4 | 1 | 2 | 0.5 | 1 | 32 | 1 | 2 |
| EX 15 | 2 | 0.5 | 1 | 0.25 | 0.5 | 32 | 0.5 | 2 |
| EX 16 | 4 | 0.5 | 1 | 0.5 | 0.5 | 8 | 0.5 | 2 |
| EX 17 | 4 | 0.5 | 1 | 0.25 | 0.5 | 8 | 0.5 | 2 |
| EX 18 | 4 | 1 | 2 | 0.5 | 1 | 32 | 2 | 4 |
| EX 19 | 2 | 2 | 2 | <0.5 | 1 | >64 | 2 | 8 |
| EX 20 | 1 | 1 | 1 | <0.5 | 1 | 64 | 1 | 4 |
| EX 21 | 4 | 1 | 2 | 1 | 2 | 64 | 2 | 4 |
| EX 22 | 8 | 4 | 4 | 2 | 4 | >64 | 4 | 8 |

### Example 1 Preparation of N{[(5S)-3-(3-fluoro-4-{4-[2-(methylsulfinyl)acetyl]-1-piperazinyl}phenyl)-2-oxo-1,3-oxazolidin-5-yl]methyl}propanethioamide (6) PNU-255889

### Step 1

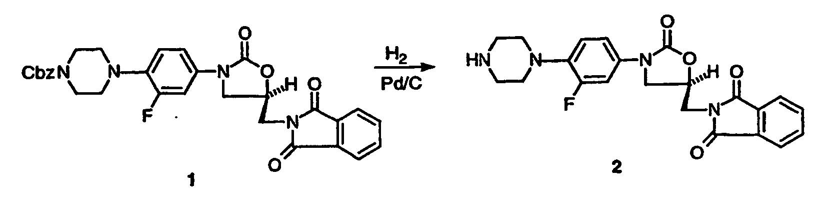

A mixture of **1** (may be prepared according to US Patent No. 5,547,950) (5.00 g, 8.95 mmol), EtOH (150 ml), THF (150 ml), concentrated hydrochloric acid (1.5 ml) and 10% palladium on carbon catalyst (2 g) is hydrogenated at an initial pressure of 32 p.s.i. for 18 hours. The mixture is filtered and the solid is washed with MeOH/CH₂Cl₂.
Concentration of the combined filtrate gave a solid which is stirred for 18 hours with a mixture of saturated aqueous NaHCO₃ (100 ml) and EtOAc (100 ml), collected by filtration washed with water and dried. It is dissolved in 20% MeOH/CH₂Cl₂, dried (MgSO₄) and concentrated to give 1.94 g of compound **2**.

### Step 2

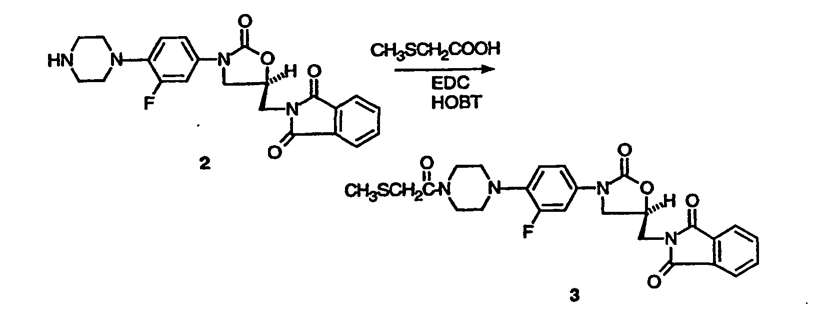

A stirred mixture of **2** (1.70 g, 4.01 mmol), 1-hydroxybenzotriazole hydrate (HOBT, 650 mg, 4.81 mmol), (methylthio)acetic acid (419 µL, 4.81 mmol) and DMF (38 ml) is cooled to 0°C and treated with 1-(3-dimethylaminopropyl)-3-ethylcarbodiimide hydrochlroride (EDC, 1.54 g, 8.02 mmol). It is kept at 0°C for two days and concentrated *in vacuo* at 50°C. The residue is mixed with water and extracted with EtOAc. The extract is dried (MgSO₄) and concentrated. Chromatography of the residue on silica gel with mixtures of MeOH/CH₂Cl₂ containing 1-2% MeOH gave 1.75 g of compound **3**.

### Step 3

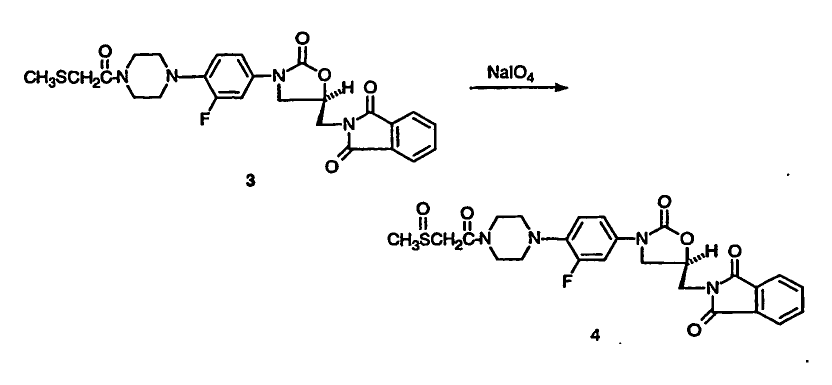

A stirred, ice cold mixture of **3** (1.70 g, 3.32 mmol) in MeOH (17 ml) and water (8.5 ml) is treated with sodium periodate (1.06 g, 4.98 mmol) and kept in the ice bath for 4 hours and at ambient temperature (24°C) for 4 days. It is concentrated *in vacuo*, mixed with water and extracted with CH₂Cl₂. The extract is dried (MgSO₄) and concentrated. Chromatography of the residue on silica gel with 5% MeOH-CH₂Cl₂ gave 1.22 g of compound **4**.

### Step 4

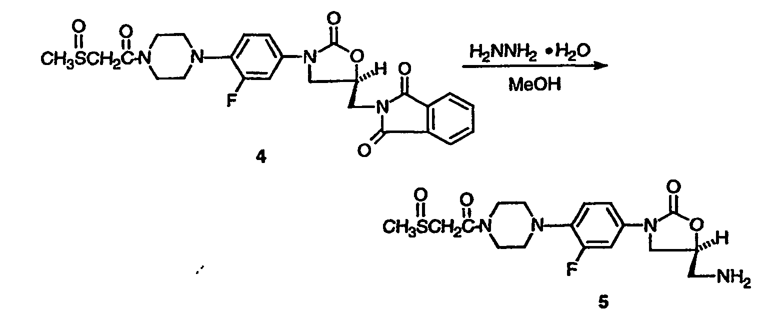

A stirred mixture of **4** (964 mg, 1.82 mmol), hydrazine hydrate (177 µL, 3.64 mmol) and MeOH (16 ml) is warmed at 80°C for 6 hours and kept at ambient temperature for 4 days. It is concentrated *in vacuo.* Chromatography of the residue on silica gel with 10% MeOH-1% NH₄OH-CH₂Cl₂ gave 630 mg of compound **5.**

### Step 5

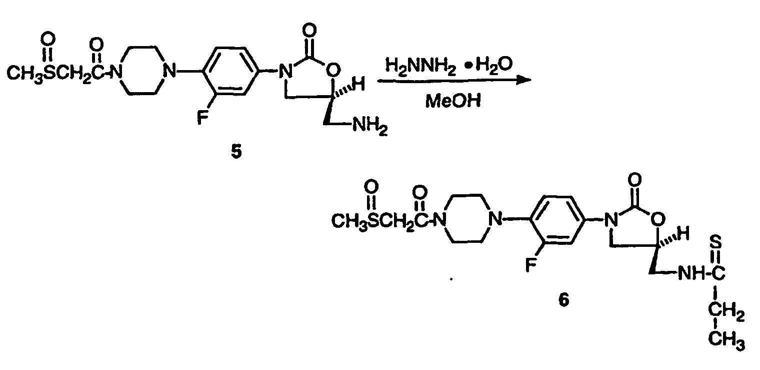

A stirred mixture of 5 (326 mg, 0.815 mmol), triethylamine (0.91 mL, 6.55 mmol), and methyl dithiopropionate (393 mg, 3.27 mmol) in CH₂Cl₂ (8.0 ml) and THF (8 ml) is kept at ambient temperature (24°C) for 18 hours, mixed with water and extracted with CH₂Cl₂. The extract is dried (MgSO₄) and concentrated. Chromatography of the residue on silica gel with mixtures of MeOH/CH₂Cl₂ containing 2.5-5% MeOH gave the product which is recrystallized from MeOH to give 257 mg of compound **6**. Anal. calcd for C₁₀H₂₇FN₄O₄S₂: C, 51.05; H, 5.78; N, 11.91. Found: C, 50.82; H, 5.85; N, 11.80.

### Example 2 Preparation of N-{[(5S)-3-(3-fluoro-4-{4-[2-(methylsulfanyl)acetyl]-1-piperazinyl}phenyl)-2-oxo-1,3-oxazolidin-5-yl]methyl}propanethioamide (10) (PNU-247827).

### Step 1

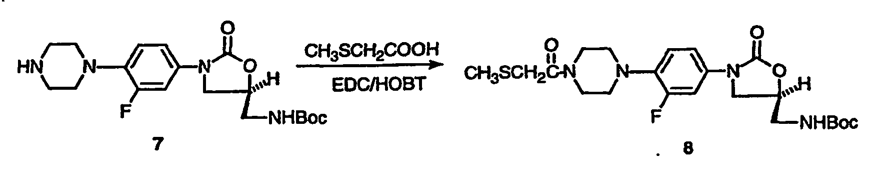

A stirred, ice cold, solution of **7** (may be prepared according to PCT International Publication WO 98/54161) (3.00 g, 7.61 mmol), HOBT (1.13 g, 2.79 mmol) and methylthioacetic acid (0.66 mL, 2.54 mmol) in DMF (69 ml) are treated with EDC (3.21 g, 5.58 mmol) and allowed to warm slowly to ambient temperature (24°C) during about 18 hours. It is concentrated *in vacuo* at 50°C and the residue is mixed with water and extracted with EtOAc. The extract is washed with water and brine, dried (MgSO₄) and concentrated. Crystallization of the residue from MeOH/EtOAc/heptane gave 2.36 g of compound **8**.

### Step 2

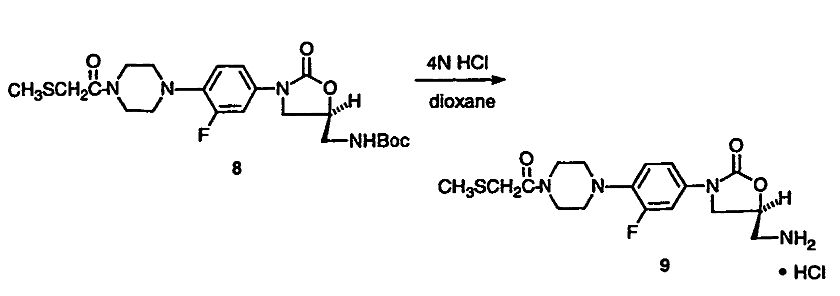

A sample of **8** (1.00 g, 2.07 mmol) is cooled in an ice bath, treated with 4N HCl in dioxane (10 ml) and stirred in the bath for 1.5 hours and at ambient temperature (24°C) for 1 hour. The mixture is concentrated and the residue is mixed with three portions of CH₂Cl₂ with concentration after each addition to give **9.**

### Step 3

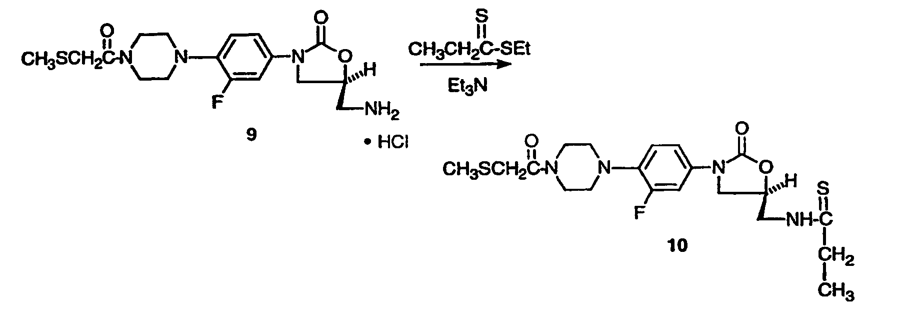

A stirred mixture of compound **9** (578 mg, 1.38 mmol), triethylamine (1.5 mL, 11.0 mmol), ethyl dithiopropionate (0.76 mL, 5.52 mmol), CH₂Cl₂ (15.5 ml) and THF (15.5 ml) is kept at ambient temperature (24°C) for 18 hours and concentrated *in vacuo.* The residue is stirred with a mixture of water (30 ml) and 10% EtOAc-heptane (30 ml) for 2 hours and the solid is collected by filtration, washed with water, dried and crystallized from EtOAc-MeOH-heptane. The resulting solid is chromatographed on silica gel with 2% MeOH-CH₂Cl₂ and the product is crystallized from MeOH/EtOAc to give 465 mg of compound **10:** MS (EI) *m*/*z* 454 (M⁺). Anal. Calcd for C₂₀H₂₇FN₄O₃S₂: C, 52.84; H, 5.99; N, 12.32. Found: C, 52.83; H, 6.02; N, 12.23.

### Example 3 Preparation of N-{[(5S)-3-(3-fluoro-4-{4-[2-(methylsulfonyl)acetyl]-1-piperazinyl}phenyl]-2-oxo-1,3-oxazolidin-5-yl]methyl}propanethioamide (13) (PNU-248337)

### Step 1

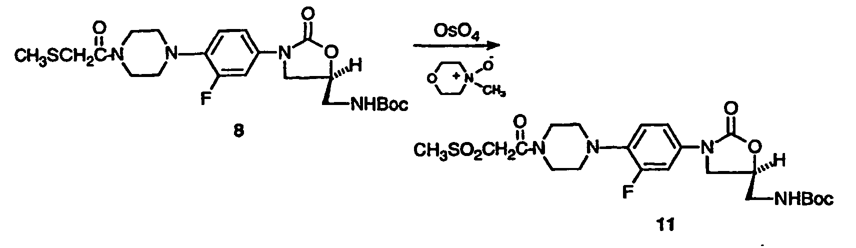

A stirred mixture of compound **8** (100 mg, 0.207 mmol), 4-methylmorpholine, N-oxide (73 mg, 0.621 mmol), acetone (1.5 ml) and water (0.5 ml) is treated with a 2.5% solution of osmium tetroxide in 2-methyl-2-propanol (17 µL) and kept at ambient temperature (24°C) for 18 hours. It is then treated with 10% aqueous NaHSO₃ (60 ml) and extracted with CH₂Cl₂. The extracts are washed with 10% NaHSO₃, dried (MgSO₄) and concentrated. Crystallization of the residue from EtOAc-heptane gave 96 mg of **11:** MS (EI) *m*/*z* 514 (M⁺).

### Step 2

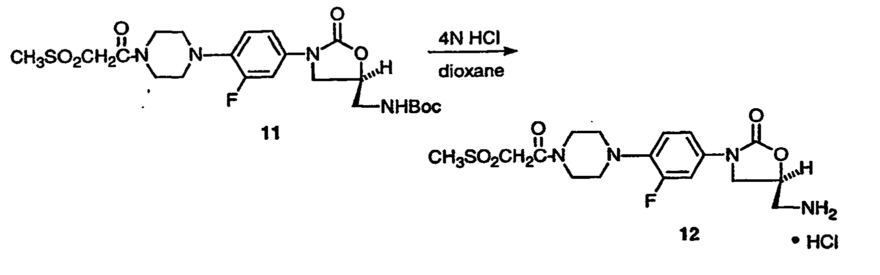

As described in Example 2, Step 2 compound **11** is treated with 4N HCl in dioxane to give **12:** HRMS (FAB) calcd for C₁₇H₂₄FN₄O₅S (M+H⁺) 415.1451, found 415.1445.

### Step 3

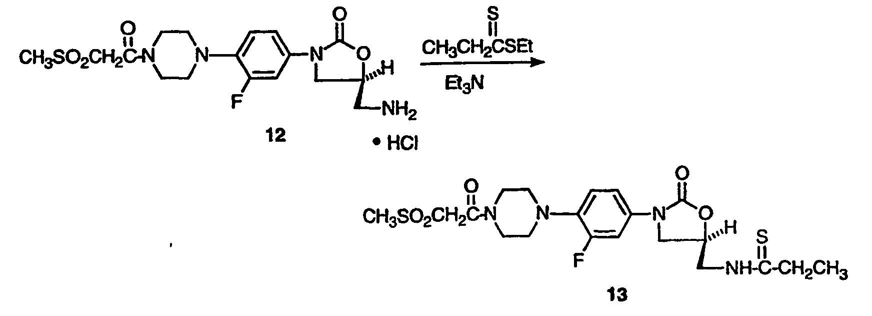

As described in Example 2, Step 3 compound **12** is allowed to react with ethyl dithiopropionate and triethylamine to give **13** which is purified by chromatography on silica gel with 1% MeOH-CH₂Cl₂ and crystallization from MeOH-EtOAc: MS(EI) *m*/*z* 486 (M⁺); HRMS (FAB) calcd for C₂₀H₂₈FN₄O₅S₂ (M+H⁺) 487.1485, found 487.1494. Anal. Calcd for C₂₀H₂₇FN₄O₅S₂: C, 49.37; H, 5.59; N, 11.51. Found: C, 49.25; H, 5.63; N, 11.47.

### Example 4 Preparation of N-({(5S)-3-[4-(4-ethanethiolyl-1-piperazinyl)-3-fluorophenyl]2-oxo-1,3-oxazolidin-5-yl}methyl)propanethioamide (15) (PNU-276575).

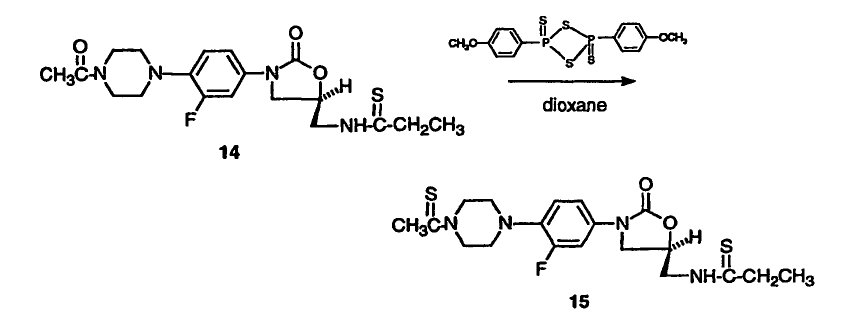

A stirred mixture of **14** ((*S*)-N-[[3-[3-fluoro-4-(4-acetyl-1-piperazinyl)phenyl]-2-oxo-5-oxazolidinyl]methyl]propanethioamide (may be prepared according to the procedure disclosed in PCT International Publication WO 98/54161) (0.53 g, 1.3 mmol), Lawesson's Reagent (0.53 g) and dioxane (27 ml) is refluxed, under nitrogen for 90 min, cooled and concentrated *in vacuo*. Chromatography of the residue on silica gel with 2% MeOH-CH₂Cl₂ gave the product which is decolorized with activated carbon and crystallized from acetonitrile to give 0.303 g of **15**: mp 209-210°C. Anal. Calcd for C₁₉H₂₅FN₄O₂S₂: C, 53.75; H, 5.93; N, 13.20. Found: C, 53.69; H, 6.00; N, 13.25.

### Example 5 Preparation of N-({(5S)-3-[4-(4-cyano-1-piperazinyl)-3-fluorophenyl]-2-oxo-1,3-oxazolidin-5-yl}methyl)propanethioamide (18) (PNU-278605)

### Step 1

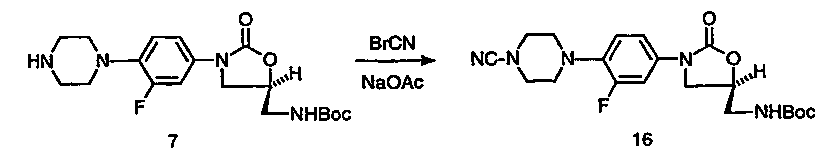

A stirred, ice cold mixture of **7** (0.488 g, 1.24 mmol) and sodium acetate (0.55 g, 6.7 mmol) in MeOH (40 ml) is treated during 1 minute, with a MeOH (10 ml) solution of 5M cyanogen bromide in CH₂Cl₂ (0.35 ml, 1.48 mmol) and kept in the ice bath for 2 hours. It is then concentrated *in vacuo* and the residue is mixed with dilute NaHCO₃ and extracted with CH₂Cl₂. The extract is washed with water, dried (Na₂SO₄) and concentrated. Chromatography of the residue on silica gel with 2% MeOH-CH₂Cl₂ gave 0.42 g of **16**: MS(ES) *m*/*z* 420 (M+H⁺).

### Step 2

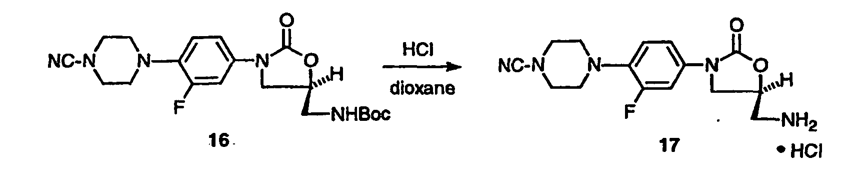

A stirred ice cold suspension of **16** (0.42 g, 1.0 mmol) in dioxane (10 ml), under nitrogen is treated dropwise with ice cold 4N HCl in dioxane (10 ml), kept in the ice bath for 2 hours and concentrated *in vacuo*. The residue is dried *in vacuo* for 18 hours to give **17:** MS(ES) *m*/*z* 320 (M+H⁺).

### Step 3

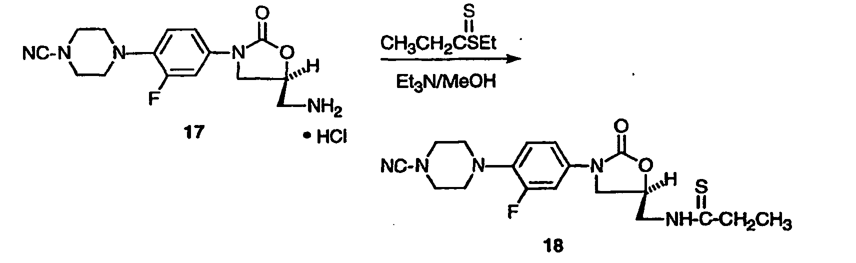

A stirred mixture of **17** (0.25 g, 0.64 mmol), and triethylamine (0.178 ml) in MeOH (5 ml), under nitrogen is warmed to 50°C during 30 minutes, kept at 50°C for 30 minutes and cooled in an ice bath. The solid is collected by filtration and crystallized from EtOH to give **18:** mp 182-184°C; HRMS (FAB) calcd for C₁₈H₂₃FN₅O₂S (M+H⁺) 392.1556, found 392.1550.

### Example 6 Preparation of N-({(5S)-3-(3-fluoro-4-{4-[2-(methylaminocarbonyloxy) acetyl]-1-piperazinyl}phenyl)-2-oxo-1,3-oxazolidin-5-yl}methyl)propanethioamide (22) (PNU-281328)

### Step 1

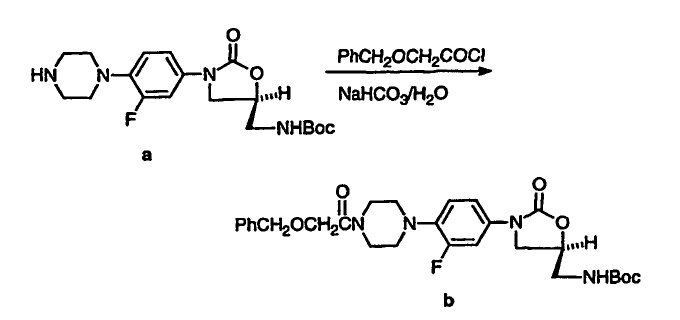

A stirred, ice cold mixture of a (PCT International Publication WO 98/54161) (20.0 g, 50.7 mmol), acetone (1500 mL) and saturated aqueous sodium bicarbonate (500 ml) is treated, during 20 min, with a solution of benzyloxyacetyl chloride (9.5 ml, 60.8 mmol) in acetone (150 ml). The mixture is allowed to warm slowly to ambient temperature (24°C) and stand for 18 hours. It is extracted with Et₂O and the extract is washed with water and brine, dried (MgSO₄), and concentrated to give 25.4 g of the product **b.**

### Step 2

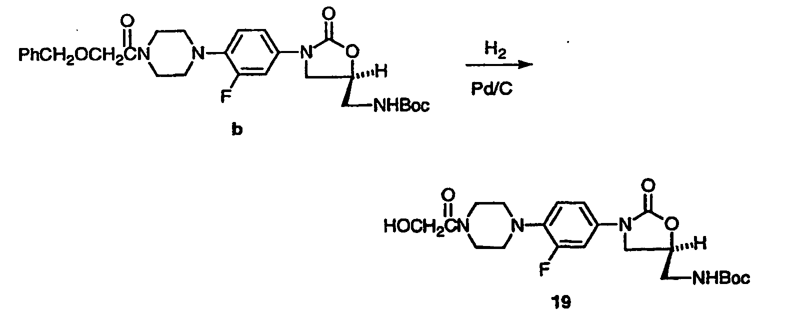

A mixture of **2** (25.0 g, 46.1 mmol), MeOH (1700 ml) and 10% palladium - on - carbon catalyst (6.25 g) is hydrogenated at an initial pressure of 35 p.s.i. for 4 days. Additional catalyst (6.25 g) is added and the hydrogenation is continued for 1 day. The mixture is filtered and the filtrate is concentrated. Chromatography of the residue on silica gel with 2.5% MeOH-CH₂Cl₂ gave the product which is crystallized from acetone - CH₂Cl₂ to give 13.7 g of **3.**

### Step 3

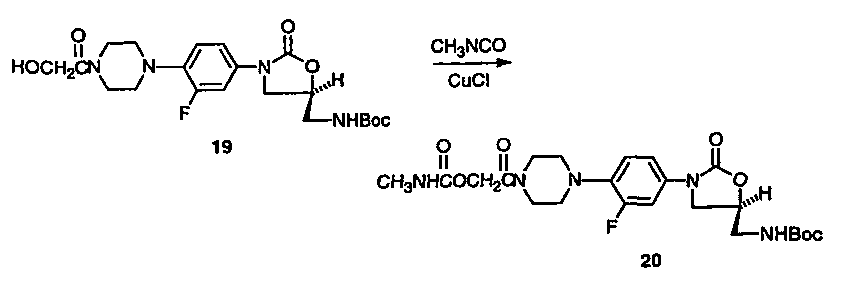

A stirred mixture of **19** and cuprous chloride (0.075 g) in DMF (4 ml) is treated with methyl isocyanate (0.081 ml), kept at ambient temperature (24°C) for 60 minutes and concentrated *in vacuo*. The residue is mixed with water and Et₂O to give a solid which is collected by filtration and chromatographed on silica gel with 2.5% MeOH-CH₂Cl₂ to give 0.28 g of **20.**

### Step 4

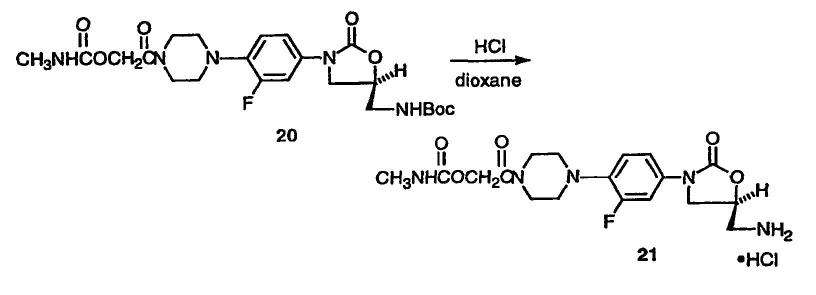

An ice cold, stirred mixture of **20** (0.37 g, 0.726 mmol) in dioxane (10 ml), under nitrogen is treated, drop-wise with ice cold 4N hydrogen chloride in dioxane (8 ml). The mixture is kept in the ice bath for 1 hour 15 minutes and at ambient temperature (24°C) for 1 hour. It is diluted with additional dioxane (10 ml), kept at ambient temperature for 30 minutes, at 0°C for 18 hours and at ambient temperature for 6 hours. It is concentrated *in vacuo* to give **21**: MS(ES) *m*/*z* 410 (M+H⁺).

### Step 5

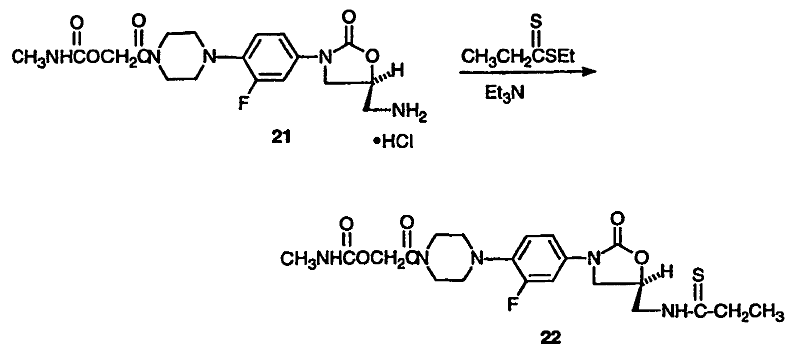

A stirred mixture of **21** (0.20 g, 0.416 mmol), ethyl dithiopropionate (0.17 ml) and triethylamine (0.5 ml) in CH₂Cl₂ (20 ml) and MeOH (5 ml) is kept, under nitrogen at ambient temperature (24°C) for 21 hours and concentrated under a stream of nitrogen. Chromatography of the residue on silica gel with 2% MeOH-CH₂Cl₂ gave **22**: HRMS (FAB) calcd for C₂₁H₂₉FN₅O₅S (M+H⁺) 482.1873, found 482.1873.

### Example 7 Preparation of N-({(5S)-3-(3-fuoro-4-{4-[2-[(2-methoxyethoxy) carbonyloxy]acetyl]-1-piperazinyl}phenyl)-2-oxo-1,3-oxazolidin-5-yl}methyl) propanethioamide (24) (PNU-276528)

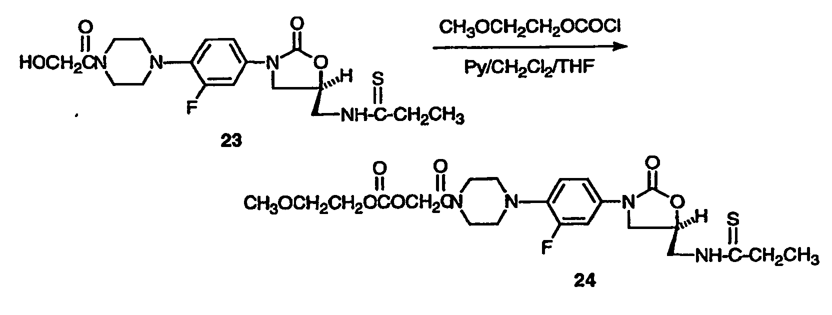

A stirred, ice cold mixture of **23** (may be prepared according to the procedure disclosed in PCT International Publication WO 98/54161) (0.212 g, 0.496 mmol) and pyridine (0.2 ml, 2.5 mmol) in CH₂Cl₂ (5 ml) and THF (5 ml) is treated, dropwise with 2-methoxyethyl chloroformate (0.069 g, 0.5 mmol) and kept in the ice bath for 1 hour and at ambient temperature (24°C) for 2 hours. Additional 2-methoxyethyl chloroformate (0.07 ml) is added; the mixture is kept at ambient temperature for 3 hours and again treated with additional 2-methoxyethyl chloroformate (0.1 ml). This mixture is kept at ambient temperature for 18 hours. It is mixed with saturated aqueous NaHCO₃ and extracted with CH₂Cl₂. The extract is washed with water and brine, dried (Na₂SO₄) and concentrated. Chromatography of the residue on silica gel with 2.5% MeOH-CH₂Cl₂ gave the product which is crystallized from EtOAc to give 0.185 g of **24:** mp 150-151°C. Anal. Calcd for C₂₃H₃₁FN₄O₇S: C, 52.46; H, 5.93; N, 10.64. Found: C, 52.45, H, 6.05; N, 10.61.

### Example 8 Preparation of N-[((5S)-3-{3-fluoro-4-[4-((2S)-2-hydroxy-3-methoxypropanoyl)-1-piperazinyl]phenyl}-2-oxo-1,3-oxazolidin-5-yl)methyl] propanethioamide (33) (PNU-272200).

### Step 1

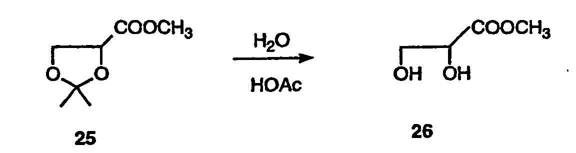

A stirred solution of methyl (*S*)-(-)-2,2-dimethyl-1,3-dioxolane-4-carboxylate (**25**) (5.0 g, 0.031 mol) in acetic acid (10 ml) and water (2.5 ml) is kept at ambient temperature for 72 hours and concentrated *in vacuo* to give (*S*)- **26**.

### Step 2

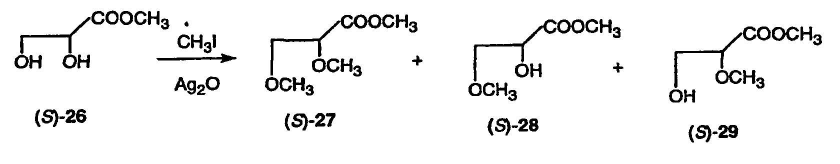

A stirred mixture of (*S*)-**26** (1.0 g) from Step 1 and methyl iodide (20 ml), under nitrogen is treated with silver oxide (1.3 g) and 3A molecular sieves (2 g) and warmed at 43°C for 90 minutes. It is cooled and filtered. The filtrate is concentrated and the residue is chromatographed on silica gel with mixtures of MeOH-CH₂Cl₂ containing 2-4% MeOH. The products eluted from the column are (*S*)-**27** (0.15 g), (*S*)-**28** (0.08 g), and (*S*)-**29** (0.28 g).

### Step 3

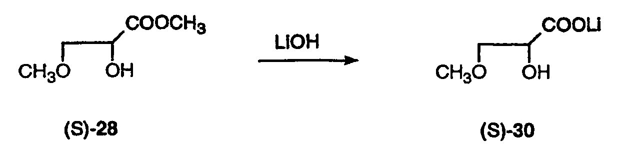

A stirred mixture of (*S*)-**28** (0.08 g, 0.6 mmol) and MeOH (3.0 ml) under nitrogen, is treated with 1M lithium hydroxide (0.57 ml) and kept at ambient temperature (24°C) for 3 hours and under a stream of nitrogen for 18 hours. It is then concentrated *in vacuo* to give (S)-**30**.

### Step 4

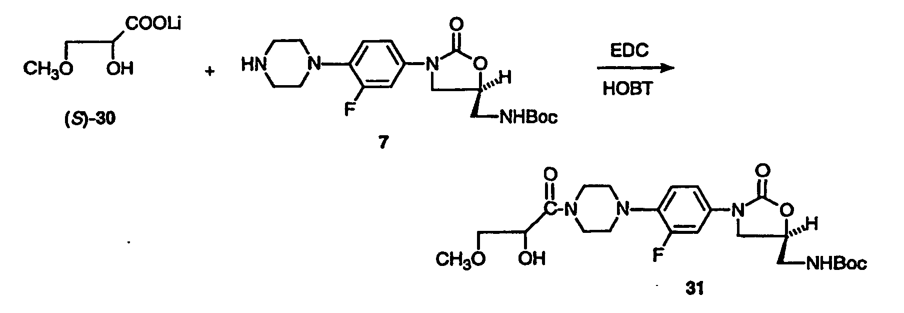

A stirred mixture of **7** (0.237 g, 0.601 mmol), the product ((*S*)-**30**) from Step 3, HOBT 0.095 g (0.703 mmol) and DMF (4 ml), under nitrogen, is treated with EDC (0.26 g, 1.36 mmol), kept at ambient temperature (24°C) for 2.5 hours and concentrated *in vacuo.* The residue is chromatographed on silica gel with 2.5% MeOH-CH₂Cl₂ to give 0.18 g of **31:** MS(ES) *m*/*z* 497 (M+H⁺).

### Step 5

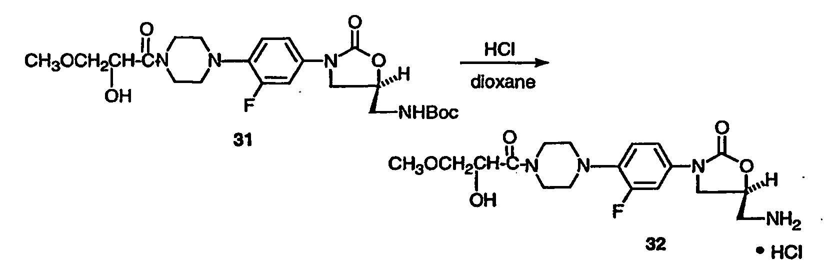

An ice cold stirred mixture of **31** (0.17 g, 0.342 mmol) in dioxane (10 ml), under nitrogen, is treated, dropwise during 3 minutes with cold 4N HCl in dioxane (10 ml) and kept in the ice bath for 50 minutes, at ambient temperature (24°C) for 90 minutes and at 0°C for 18 hours. It is then concentrated *in vacuo* to give **32:** MS(ES) *m*/*z* 397 (M+H⁺).

### Step 6

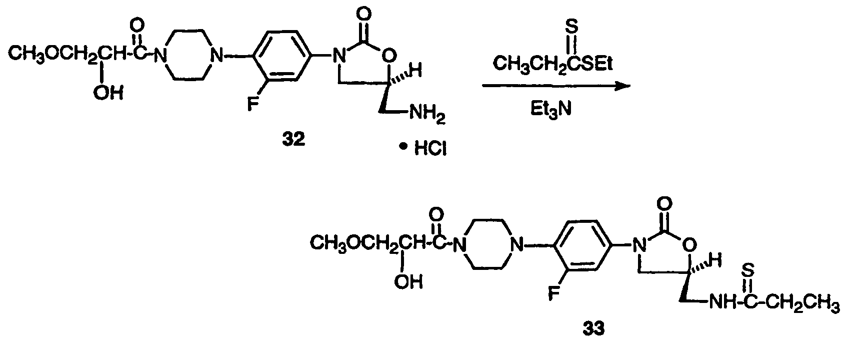

A stirred mixture of **32** from Step 5, triethylamine (0.5 ml, 3.5 mmol), CH₂Cl₂ (10 ml and THF (7 ml), under nitrogen is treated, dropwise with ethyl dithiopropionate (0.22 ml, 1.71 mmol) and kept at ambient temperature (24°C) for 72 hours. Additional ethyl dithiopropionate (0.22 ml) is added and the mixture is kept at ambient temperature for 24 hours and concentrated. The residue which still contain **32** is mixed with CH₂Cl₂ (10 ml), THF (7 ml), triethylamine (0.75 ml) and ethyl dithiopropionate (0.35 ml), kept at ambient temperature for 24 hours and concentrated. Chromatography of the residue on silica gel with 2.5% MeOH-CH₂Cl₂ gave 0.0457 g of **33:** mp 190-191°C (dec). Anal. Calcd for C₂₁H₂₉FN₄O₅S: C, 53.83; H, 6.24; N, 11.96. Found: C, 53.59; H, 6.35; N, 11.83.

### Example 9 Preparation of N-[((5S)-3-{3-fluoro-4-[4-((2S)-2,3-dimethyoxypropanoyl)-1-piperazinyl]phenyl}-2-oxo-1,3-oxazolidin-5-yl)methyl]propanethioamide (34) (PNU-272199)

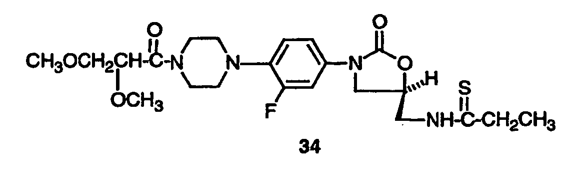

As described in Example 8 the ester ((*S*)-**27**, prepared in Step 2) is hydrolyzed with lithium hydroxide and coupled with **7.** The resulting amide is deprotected and allowed to react with ethyl dithiopropionate and triethylamine. The product is purified by silica gel chromatography with 2.5% MeOH-CH₂Cl₂ and crystallized form EtOAc-hexane to give **34:** mp 140-142 °C (dec). Anal. calcd for C₂₂H₃₁FN₄O₅S: C, 54.76; H, 6.47; N, 11.61. Found: C, 54.53; H, 6.54; N, 11.50.

### Example 10 Preparation of N-[((5S)-3-{3-fluoro-4-[4-((2S)-3-hydroxy-2-methyoxypropanoyl)-1-piperazinyl]phenyl}-2-oxo-1,3-oxazolidin-5-yl)methyl] propanethioamide (35) (PNU-272198)

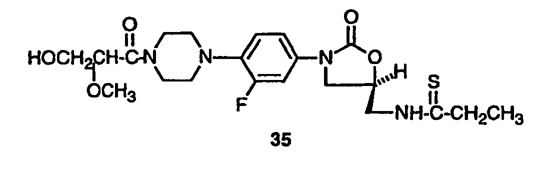

As described in Example 8 the ester ((*S*)-29, prepared in Step 2) is hydrolyzed with lithium hydroxide and coupled with **7**. The resulting amide is deprotected and condensed with ethyl dithiopropionate. The product is purified by silica gel chromatography with 2.5% MeOH-CH₂Cl₂ to give 35. Anal. calcd for C₂₁H₂₉FN₄O₅S: C, 53.83; H, 6.24; N, 11.96. Found: C, 53.71; H, 6.32; N, 11.85.

### Example 11 Preparation of N-({(5S)-3-[3-fluoro-4-(4-acetoacetyl-1-piperazinyl)phenyl]-2-oxo-1,3-oxazolidin-5-yl}methyl)propanethioamide (36)

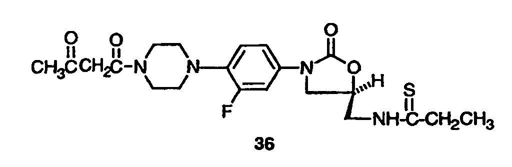

As described in Example 8 (Steps 4-6) the lithium salt of acetylacetic acid is coupled with 7 and the resulting amide is deprotected and allowed to react with ethyl dithiopropionate and triethylamine. The product is purified by silica gel chromatography to give **36:** MS(ES) *m*/*z* 451 (M+H⁺), 473 (M+Na⁺).

### Example 12 Preparation of N-({(5S)-3-[3-fluoro-4-(4-pyruvoyl-1-piperazinyl)phenyl]-2-oxo-1,3-oxazolidin-5-yl}methyl)propanethioamide (37) PNU-264886

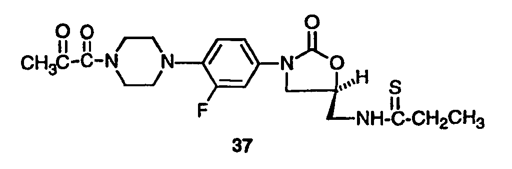

As described in Example 8 (Steps 4-6) pyruvic acid is coupled with **7** and the resulting amide is deprotected and allowed to react with ethyl dithiopropionate and triethylamine. The product is purified by silica gel chromatography with 2% MeOH-CH₂Cl₂ and crystallized from EtOAc-hexane to give **37:** mp 173-175 °C (dec); MS(ES) *m*/*z* 437 (M+H⁺), 459 (M+Na⁺). Anal. calcd for C₂₀H₂₅FN₄O₄S·0.1 EtOAc; C, 54.97; H, 5.84; N, 12.57. Found: C, 55.05; H, 6.15; N, 12.12

### Example 13 Preparation of N-({(5S)-3-[3-fluoro-4-[4-(3-hydroxypropanoyl)-1-piperazinyl]phenyl]-2-oxo-1,3-oxazolidin-5-yl}methyl]propanethioamide (43) (PNU-272690)

### Step 1

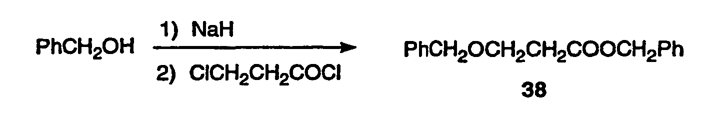

An ice cold, stirred solution of benzyl alcohol (4.0 mL, 0.0386 mol) in THF (20 mL), under nitrogen is treated, portionwise during 40 minutes with a 60% oil dispersion of sodium hydride (1.6 g, 0.04 mol), kept in the ice bath for 20 minutes and treated during 5 minutes with a solution of 3-chloropropionyl chloride (1.50 mL, 0.0157 mol) in THF (3 mL). The mixture is warmed slowly to ambient temperature (24 °C), kept for 23 hours, mixed with saturated ammonium chloride (15 mL) and ice water and extracted with EtOAc. The extract is washed with water and brine, dried (Na₂SO₄) and concentrated to give **38:** MS(ES) *m*/*z* 293 (M+Na⁺).

### Step 2

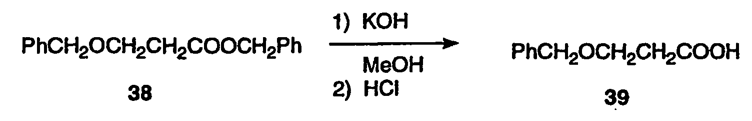

An ice cold, stirred solution of **38** from Step 1 in MeOH (50 mL), under nitrogen, is treated with potassium hydroxide (0.94 g, 0.0168 mol) and kept in the ice bath for 10 minutes, at ambient temperature for 1 hour and at -20 °C for 18 hours. It is treated with additional potassium hydroxide (0.98 g), kept at ambient temperature for 8.5 hours and at -20 °C for 18 hours and concentrated *in vacuo*. The residue is mixed with ice water, cooled in an ice bath and treated with 2N HCl to pH 3. It is extracted with EtOAc. The extract is washed with 2N NaOH and water and the wash is reacidified with 2N HCl and extracted with EtOAc. The extract is concentrated to give 1.48 g of **39:** MS(ES) *m*/*z* 181 (M+H⁺), 203 (M+Na⁺).

### Step 3

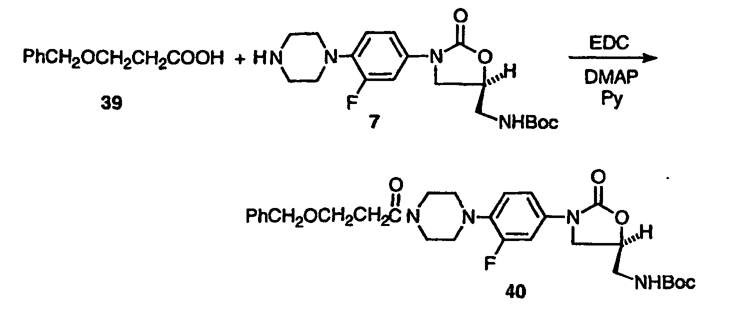

A stirred mixture of **7** (0.5 g, 1.26 mmol) and pyridine (6 mL), under nitrogen, is treated with 4-(dimethylamino)pyridine (DMAP, 8 mg), EDC (0.243 g, 1.26 mmol) and a solution of **39** (0.228 g, 1.26 mmol) in CH₂Cl₂ (2mL) and kept at ambient temperature (24 °C) for 2 hours 20 minutes. It is concentrated *in vacuo* and the residue is mixed with CH₂Cl₂, washed with saturated NaHCO₃, water and brine, dried (Na₂SO₄) and concentrated. Chromatography of the residue on silica gel with 2.5% MeOH-CH₂Cl₂ gave 0.43 g of **40:** MS(ES) *m*/*z* 557 (M+H⁺), 579 (M+Na⁺).

### Step 4

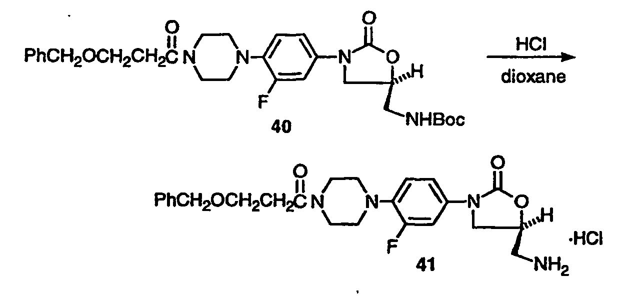

A stirred, ice cold solution of **40** (0.43 g, 0.772 mmol) in dioxane (12 mL), under nitrogen is treated with 4N hydrogen chloride in dioxane (10 mL), dropwise during 3 minutes. It is warmed to ambient temperature (24 °C) during 90 minutes, kept for 3 hours 30 minutes and concentrated to give 0.43 g of **41**.

### Step 5

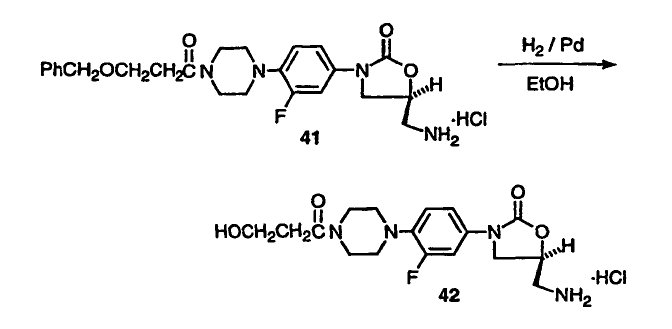

A mixture of **41** (0.21 g), 10% palladium on carbon catalyst (0.17 g) and EtOH (50 mL) is hydrogenated at an initial pressure of 44 p.s.i. for 90 minutes, treated with additional catalyst (0.1 g) and hydrogenated at an initial pressure of 40 p.s.i. for 22 hours. It is filtered and the solid is washed with MeOH. The filtrates are concentrated and the residue is chromatographed on silica gel with mixtures of MeOH-NH₄OH-CH₂Cl₂ that contained 5-7.5% MeOH and 0.25-0.5% NH₄OH to give 0.07 g of 42: MS(ES) *m*/*z* 367 (M+H⁺).

### Step 6

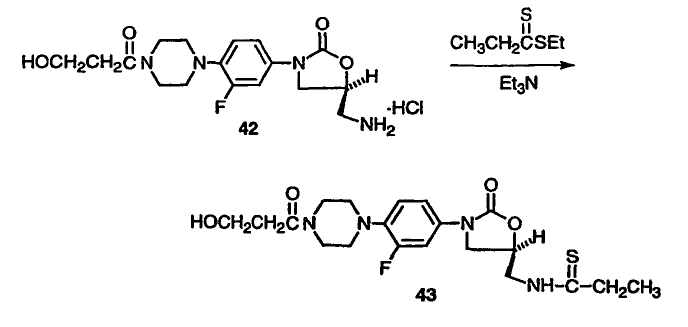

A stirred mixture of **42** (0.07 g, 0.19 mmol), CH₂Cl₂ (8 mL) and THF (8 mL) is treated with triethylamine (0.20 mL) and ethyl dithiopropionate (0.08 mL) and kept at ambient temperature (24 °C) for 24 hours, at 45 °C for 7.5 hours and at ambient temperature for 16 hours. It is then concentrated and the residue is chromatographed on silica gel with mixtures of MeOH-CH₂Cl₂ that contained 2-3.5% MeOH. The product (**43**) amounted to 0.057 g: HRMS (FAB) calcd for C₂₀H₂₈FN₄O₄S (M+H⁺) 439.1815, found 439.1812.

### Example 14 Preparation of N-{[(5S)-3-(3-fluoro-4-{4-[(1-hydroxycyclopropyl)carbonyl]-1-piperazinyl}phenyl)-2-oxo-1,3-oxazolidin-5-yl]methyl}propanethioamide (44) (PNU-251110)

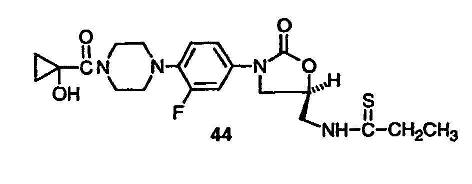

As described in Example 8 (Steps 4-6) 1-hydroxy-1-cyclopropanecarboxylic acid is coupled with **7** and the resulting amide is deprotected and allowed to react with ethyl dithiopropionate and triethylamine. The product is purified by silica gel chromatography with mixtures of MeOH-CH₂Cl₂ that contained 2-12% MeOH and by crystallization from MeOH-EtOAc to give **44:** mp 185-186 °C (dec); MS(ES) *m*/*z* 451 (M+H⁺), 473 (M+Na⁺). Anal. calcd for C₂₁H₂₇FN₄O₄S: C, 55.99; H, 6.04; N, 12.44. Found: C, 55.78; H, 6.09; N, 12.18.

### Example 15 Preparation of N-[((5S)-3-{3-fluoro-4-[4-(2-phenoxyacetyl)-1-piperazinyl]phenyl}-2-oxo-1,3-oxazolidin-5-yl)methyl]propanethioamide (47) PNU-251037)

### Step 1

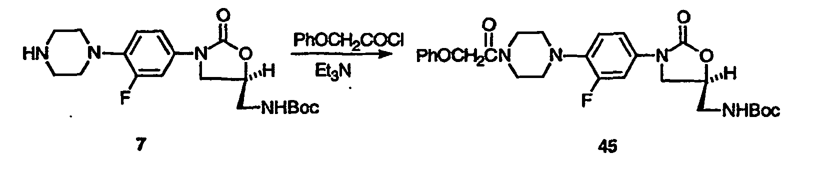

An ice cold, stirred solution of **7** (0.5 g, 1.26 mmol) and triethylamine (0.385 ml, 2.76 mmol) in CH₂Cl₂ (25 ml), under nitrogen, is treated dropwise with a solution of phenoxyacetyl chloride (0.35 ml, 2.52 mmol) in CH₂Cl₂ (3 ml) and kept in the ice bath for 2 hours and at ambient temperature for 30 minutes. It is diluted with CH₂Cl₂, washed with saturated NaHCO₃, water and brine, dried (Na₂SO₄) and concentrated. Crystallization of the residue from MeOH-EtOAc gave 0.53 g of **45:** MS(ES) *m*/*z* 529 (M+H⁺), 551 (M+Na⁺).

### Step 2

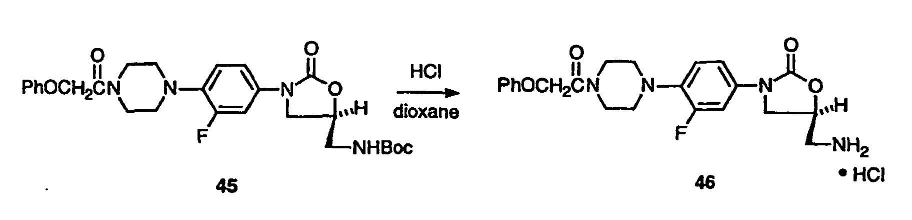

As described in Example 8 (Step 5) compound **45** is deprotected with hydrogen chloride in dioxane to give **46:** MS(ES) *m*/*z* 429 (M+H⁺).

### Step 3

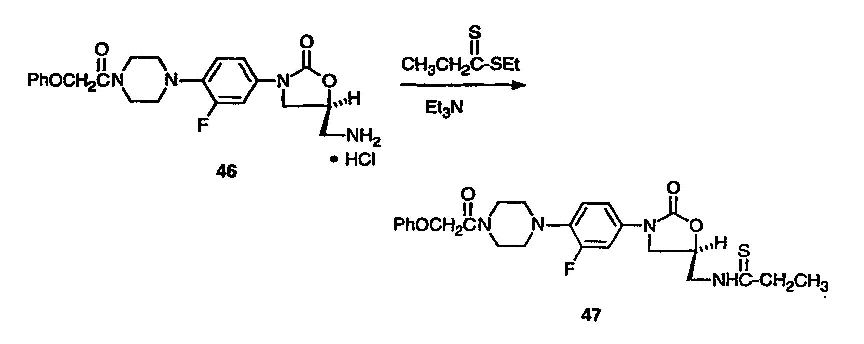

As described in Example 8 (Step 6) the amine hydrochloride (**46**) is allowed to react with ethyl dithiopropionate and triethylamine in CH₂Cl₂-THF. The product is chromatographed on silica gel with 2.5% MeOH-CH₂Cl₂ and crystallized from EtOAc to give **47:** mp 171-172°C; MS(ES) *m*/*z* 501 (M+H⁺), 523 (M+Na⁺). Anal. Calcd for C₂₅H₂₉FN₄O₄S: C, 59.98; H, 5.84; N, 11.19. Found: C, 59.59; H, 5.89; N, 11.03.

### Example 16 Preparation of N-({(5S)-3-[3-fluoro-4-[4-((2S)-2,3-dihydroxypropanoyl)-1-piperazinyl]phenyl]-2-oxo-1,3-oxazolidin-5-yl}methyl)propanethioamide (48) (PNU-248440)

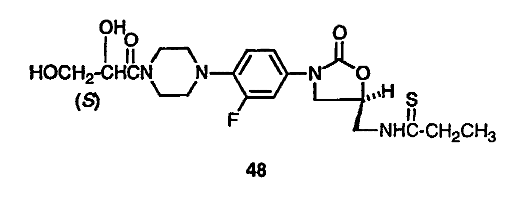

As described in Example 8 (Steps 4-6) L-glyceric acid, calcium salt dihydrate is coupled with **7** and the resulting amide is deprotected and allowed to react with ethyl dithiopropionate and triethylamine. The product is purified by silica gel chromatography with 7.5% MeOH-EtOAc to give **48:** mp 142°C (dec); MS(ES) *m*/*z* 455 (M+H⁺), 477 (M+Na⁺). Anal. Calcd for C₂₀H₂₇FN₄O₅S .0.3 EtOAc: C, 52.94; H, 6.15; N, 11.65, Found: C, 52.75; H, 6.02; N, 11.53.

### Example 17 Preparation of N-({(5S)-3-[3-fluoro-4-[4-((2R)-2,3-dihydroxypropanoyl)-1-piperazinyl]phenyl]-2-oxo-1,3-oxazolidin-5-yl}methyl)propanethioamide (49) (PNU-248438)

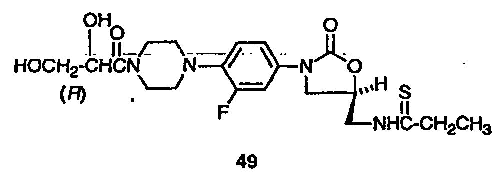

As described in Example 8 (Steps 4-6) D-glyceric acid, calcium salt dihydrate is coupled with **7** and the resulting amide is deprotected and allowed to react with ethyl dithiopropionate and triethylamine. The product is purified by silica gel chromatography with 7.5% MeOH-CH₂Cl₂ and crystallized from EtOAc-hexane to give **49:** mp 132°C (dec); MS(ES) *m*/*z* 455 (M+H⁺), 477 (M+Na⁺). Anal. Calcd for C₂₀H₂₇FN₄O₅S . 0.5 H₂O: C, 51.88; H, 6.09; N, 12.09; H₂O, 3.88. Found: C, 51.77; H, 6.09; N, 11.96; H₂O, 3.85.

### Example 18 Preparation of N-{[(5S)-3-(3-fluoro-4-{4-[3-hydroxy-2-(hydroxymethyl)-2-methylpropanoyl]-1-piperazinyl}phenyl)-2-oxo-1,3-oxazolidin-5-yl]methyl}propanethioamide (50) (PNU-248437)

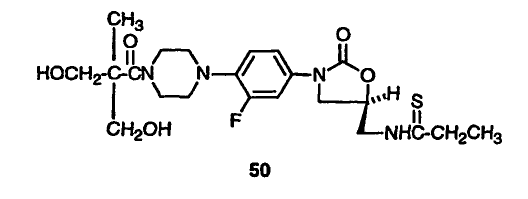

As described in Example 8 (Steps 4-6) 2,2-bis(hydroxymethyl)propionic acid is coupled with **7** and the resulting amide is deprotected and allowed to react with ethyl dithiopropionate and triethylamine. The product is purified by silica gel chromatography with 5% MeOH-CH₂Cl₂ and crystallized from MeOH-EtOAc-hexane to give **50:** mp 202-203°C (dec); MS(ES) *m*/*z* 483 (M+H⁺), 502 (M+Na⁺). Anal. Calcd for C₂₂H₃₁FN₄O₅S: C, 54.76; H, 6.47; N, 11.61. Found: C, 54.38; H, 6.54; N, 11.43.

### Example 19 Preparation of N-[((5S)-3-{3-fluoro-4-[4-((2S)-2-hydroxy-3-phenylpropanoyl)-1-piperazinyl]phenyl}-2-oxo-1,3-oxazolidin-5-yl)methyl] propanethioamide (51) (PNU-246967)

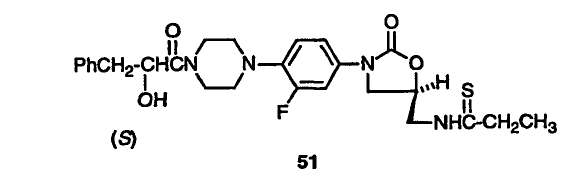

As described in Example 8 (Steps 4-6) L-3-phenyllacetic acid is coupled with **7** and the resulting amide is deprotected and allowed to react with ethyl dithiopropionate and triethylamine. The product is purified by silica gel chromatography with 2.5% MeOH-CH₂Cl₂ and crystallized from EtOAc-hexane to give 51: mp 174-175°C. Anal calcd for C₂₆H₃₁FN₄O₄S: C, 60.68; H, 6.07; N, 10.89. Found: C, 60.56; H, 6.17; N, 10.68.

### Example 20 Preparation of N-[((5S)-3-{3-fluoro-4-[4-((2R)-2-hydroxy-3-phenylpropanoyl)-1-piperazinyl]phenyl}-2-oxo-1,3-oxazolidin-5-yl) methyl]propanethioamide (52) (PNU-246966)

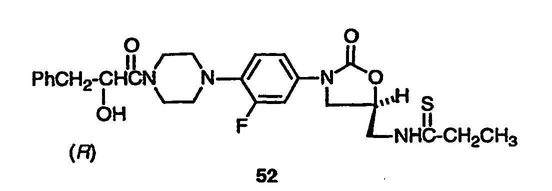

As described in Example 8 (Steps 4-6) D-3-phenyllactic acid is coupled with 7 and the resulting amide is deprotected and allowed to react with ethyl dithiopropionate and triethylamine. The product is purified by silica gel chromatography with 2.5 % MeOH-CH₂Cl₂ and crystallized from EtOAc-hexane to give **52**: mp 128-130°C (dec). Anal. Calcd for C₂₆H₃₁FN₄O₄S: C, 60.68; H, 6.07; N, 10.98. Found: C, 60.50; H, 6.17; N, 10.80.

### Example 21 Preparation of N-[((5S)-3-{3-fluoro-4-[4-((2R)-2-hydroxy-2-phenylacetyl)-1-piperazinyl]phenyl}-2-oxo-1,3-oxazolidin-5-yl)methyl]propanethioamide (53) (PNU-245689)

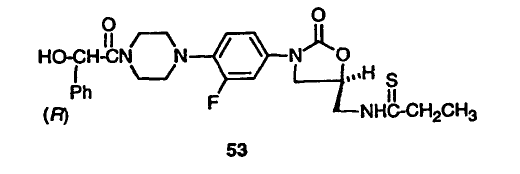

As described in Example 8 (Steps 4-6) (*R*)-(-)-mandelic acid is coupled with 7 and the resulting amide is deprotected and allowed to react with ethyl dithiopropionate and triethylamine. The product is purified by silica gel chromatography with mixtures of MeOH-CH₂Cl₂ containing 2-3.5 % MeOH to give **53**: HRMS (FAB) calcd for C₂₅H₃₀FN₄O₄S (M+H⁺) 501.1971, found: 501.1980.

### Example 22 Preparation of N-[((5S)-3-{3-fluoro-4-[4-((2S)-2-acetoxy-2-phenylacetyl)-1-piperazinyl]phenyl}-2-oxo-1,3-oxazolidin-5-yl)methyl]propanethioamide (54) (PNU-245878)

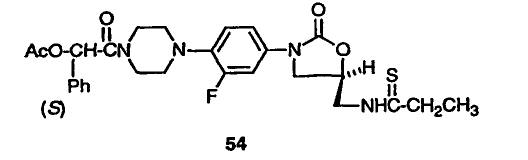

As described in Example 8 (Steps 4-6) (*S*)-(+)-O-acetylmandelic acid is coupled with **7** and the resulting amide is deprotected and allowed to react with ethyl dithiopropionate and triethylamine. The product is purified by silica gel chromatography with 2% MeOH-CH₂Cl₂ to give **54:** HRMS (FAB) calcd for C₂₇H₃₂FN₄O₅S (M+H⁺) 543.2077, found: 543.2063.

## Claims

1. A compound of formula I 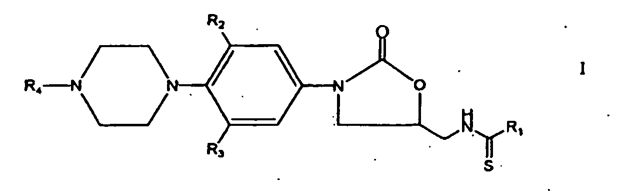 or a pharmaceutically acceptable salt thereof, wherein
R₁ is H, C₂₋₄ alkenyl, -(CH₂)₀₋₂-C₃₋₆ cycloalkyl, or C₁₋₄ alkyl optionally substituted with 1-3 F, 1-2 Cl or CN;
R₂ and R₃ are independently H, F, Cl or C₁₋₂ alkyl; and
R₄ is
(a) -CO-CHPh-O-C₁₋₄alkanoyl,
(b) -CO-CH₂-S(O)₀₋₂-CH₃,
(c) -CS-C₁₋₄ alkyl,
(d) -CO-CH₂-O-CO₂-(CH₂)₂-OCH₃,
(e) -CO-CH₂-O-CO-NH-C₁₋₃ alkyl,
(f) -CO-(CH₂)₀₋₁-CO-CH₃, or
(g) -CN.

2. A compound according to claim 1, which has the optical configuration of formula la

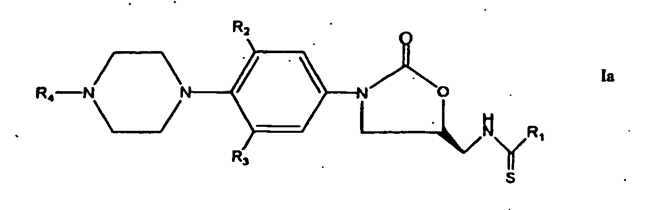

3. A compound according to claim 1 or claim 2, wherein R₁ is C₁₋₄ alkyl.

4. A compound according to claim 3, wherein R₁ is ethyl.

5. A compound according to any preceding claim, wherein R₂ and R₃ are independently H or F.

6. A compound according to claim 5, wherein R₂ is H and R₃ is F.

7. A compound of claim 1, which is
N{[(5*S*)-3-(3-fluoro-4-{4-[2-(methylsulfinyl)acetyl]-1-piperazinyl}phenyl)-2-oxo-1,3-oxazolidin-5-yl]methyl}propanethioamide,
N-{[(5*S*)-3-(3-fluoro-4-{4-[2-(methylsulfanyl)acetyl]-1-piperazinyl}phenyl)-2-oxo-1,3-oxazolidin-5-yl]methyl}propanethioamide, or
N-{[(5*S*)-3-(3-fluoro-4-{4-[2-(methylsulfonyl)acetyl]-1-piperazinyl}phenyl]-2-oxo-1,3-oxazolidin-5-yl]methyl}propanethioamide.

8. A compound of claim 1, which is N-({(5*S*)-3-[4-(4-cyano-1-piperazinyl)-3-fluorophenyl]-2-oxo-1,3-oxazolidin-5-yl}methyl)propanethioamide.

9. A compound of claim 1, which is
N-({(5*S*)-3-(3-fluoro-4-{4-[2-(methylaminocarbonyloxy)acetyl]-1-piperazinyl}phenyl)-2-oxo-1,3-oxazolidin-5-yl}methyl)propanethioamide,
N-({(5*S*)-3-(3-fluoro-4-{4-[2-[(2-methoxyethoxy)carbonyloxy]acetyl]-1-piperazinyl}phenyl)-2-oxo-1,3-oxazolidin-5-yl}methyl)propanethioamide, or
N-({(5*S*)-3-(3-fluoro-4-{4-acetoacetyl-1-piperazinyl)phenyl]-2-oxo-1,3-oxazolidin-5-yl}methyl)propanethioamide.

10. A compound of claim 1, which is N-({(5*S*)-3-[3-fluoro-4-(4-pyruvoyl-1-piperazinyl)phenyl]-2-oxo-1,3-oxazolidin-5-yl}methyl)propanethioamide.

11. A compound of claim 1, which is N-({(5*S*)-3-[4-(4-ethanethiolyl-1-piperazinyl)-3-fluorophenyl]-2-oxo-1,3-oxazolidin-5-yl}methyl)propanethioamide.

12. A compound of claim 1, which is N-[((5*S*)-3-{3-fluoro-4-[4-((2*S*)-2-acetoxy-2-phenylacetyl)-1-piperazinyl]phenyl}-2-oxo-1,3-oxazolidin-5-yl)methyl]-propanethioamide.

13. A pharmaceutical composition comprising a compound according to any preceding claim and a pharmaceutically acceptable carrier.

14. Use of a compound according to any of claim 1 to 12 for the manufacture of a medicament for treating a microbial infection in a mammal.

15. The use of claim 14, wherein the medicament is to be administered orally, parenterally, transdermally or topically.

16. The use of claim 14 or claim 15, wherein the compound is to be administered in an amount of 0.1 to 100 mg/kg of the mammal's body weight/day.

17. The use of claim 16, wherein the amount is 1 to 50 mg/kg of the mammal's body weight/day.

18. The use of any of claims 14 to 17, wherein the infection is a skin infection.

19. The use of any of claims 14 to 17, wherein the infection is an eye infection.

## Patentansprüche

1. Verbindung der Formel I 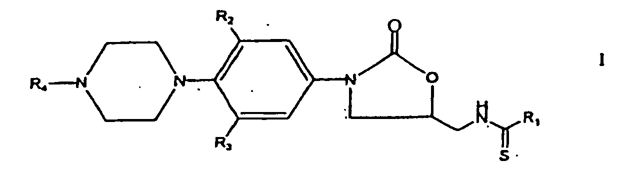 oder ein pharmazeutisch akzeptables Salz derselben, worin
R₁ H, C₂₋₄-Alkenyl, -(CH₂)₀₋₂-C₃₋₆-Cycloalkyl oder C₁₋₄-Alkyl, das optional mit 1-3 F, 1-2 Cl oder CN substituiert ist, bedeutet;
R₂ und R₃ unabhängig voneinander H, F, Cl oder C₁₋₂-Alkyl bedeuten; und
R₄
(a) -CO-CHPh-O-C₁₋₄-Alkanoyl,
(b) -CO-CH₂-S-(O)₀₋₂-CH₃,
(c) -CS-C₁₋₄-Alkyl,
(d) -CO-CH₂-O-CO₂-(CH₂)₂-OCH₃,
(e) -CO-CH₂-O-CO-NH-C₁₋₃-Alkyl,
(f) -CO-(CH₂)₀₋₁-CO-CH₃ oder
(g) -CN
bedeutet.

2. Verbindung nach Anspruch 1, die die optische Konfiguration der Formel Ia 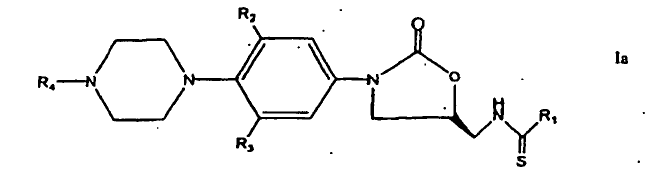 besitzt.

3. Verbindung nach Anspruch 1 oder 2, worin R₁ C₁₋₄-Alkyl ist.

4. Verbindung nach Anspruch 3, worin R₁ Ethyl ist.

5. Verbindung nach einem der vorhergehenden Ansprüche, worin R₂ und R₃ unabhängig voneinander H oder F sind.

6. Verbindung nach Anspruch 5, worin R₂ H ist und R₃ F ist.

7. Verbindung nach Anspruch 1, nämlich
N-{[(5S)-3-(3-Fluor-4-{4-[2-(methylsulfinyl)acetyl]-1-piperazinyl}phenyl)-2-oxo-1,3-oxazolidin-5-yl]methyl}propanthioamid,
N-{[(5S)-3-(3-Fluor-4-{4-[2-(methylsulfanyl)acetyl]-1-piperazinyl}phenyl)-2-oxo-1,3-oxazolidin-5-yl]methyl}propanthioamid oder
N-{[(5S)-3-(3-Fluor-4-{4-[2-(methylsulfonyl)acetyl]-1-piperazinyl}phenyl)-2-oxo-1,3-oxazolidin-5-yl]methyl}propanthioamid.

8. Verbindung nach Anspruch 1, nämlich N-({(5S)-3-[4-(4-cyano-1-piperazinyl)-3-fluorphenyl]-2-oxo-1,3-oxazolidin-5-yl}methyl)propanthioamid.

9. Verbindung nach Anspruch 1, nämlich N-({(5S)-3-(3-Fluor-4-{4-[2-(methylaminocarbonyloxy)acetyl]-1-piperazinyl}phenyl)-2-oxo-1,3-oxazolidin-5-yl}methyl)propanthioamid, N-({(5S)-3-(3-Fluor-4-{4-[2-[(2-methoxyethoxy)carbonyloxy]-acetyl]-1-piperazinyl}phenyl)-2-oxo-1,3-oxazolidin-5-yl}methyl)propanthioamid oder N-({(5S)-3-(3-Fluor-4-{4-acetoacetyl-1-piperazinyl)phenyl]-2-oxo-1,3-oxazolidin-5-yl}methyl)propanthioamid.

10. Verbindung nach Anspruch 1, nämlich N-({(5S)-3-[3-Fluor-4-(4-pyruvoyl-1-piperazinyl)phenyl]-2-oxo-1,3-oxazolidin-5-yl}methyl)propanthioamid.

11. Verbindung nach Anspruch 1, nämlich N-({(5S)-3-[4-(4-Ethanthiolyl-1-piperazinyl)-3-fluorphenyl]-2-oxo-1,3-oxazolidin-5-yl}methyl)propanthioamid.

12. Verbindung nach Anspruch 1, nämlich N-[((5S)-3-{3-Fluor-4-[4-((2S)-2-acetoxy-2-phenylacetyl)-1-piperazinyl]-phenyl}-2-oxo-1,3-oxazolidin-5-yl)methyl]propanthioamid.

13. Pharmazeutische Zusammensetzung, die eine Verbindung nach einem der vorhergehenden Ansprüche und einen pharmazeutisch akzeptablen Träger umfasst.

14. Verwendung einer Verbindung nach einem der Ansprüche 1 bis 12 zur Herstellung eines Medikaments zur Behandlung einer mikrobiellen Infektion bei einem Säuger.

15. Verwendung nach Anspruch 14, wobei das Medikament oral, parenteral, transdermal oder topisch zu verabreichen ist.

16. Verwendung nach Anspruch 14 oder Anspruch 15, wobei die Verbindung in einer Menge von 0,1 bis 100 mg/kg des Körpergewichts des Säugers/Tag zu verabreichen ist.

17. Verwendung nach Anspruch 16, wobei die Menge 1 bis 50 mg/kg des Körpergewichts des Säugers/Tag beträgt.

18. Verwendung nach einem der Ansprüche 14 bis 17, wobei die Infektion eine Hautinfektion ist.

19. Verwendung nach einem der Ansprüche 14 bis 17, wobei die Infektion eine Augeninfektion ist.

## Revendications

1. Composé de formule I 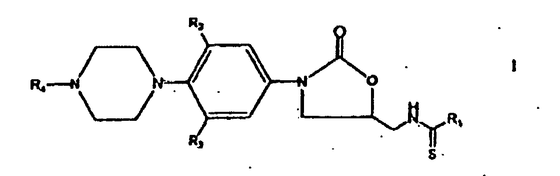 où un de ses sels pharmaceutiquement acceptables, formule dans laquelle
R₁ représente H, un groupe alcényle en C₂ à C₄, un groupe -CH₂₀₋₂ (cycloalkyle en C₃ à C₆), ou un groupe alkyle en C₁ à C₄ facultativement substitué avec un à trois substituants F, un ou 2 substituant Cl ou CN ;
R₂ et R₃ représentent indépendamment H, F, Cl ou un groupe alkyle en C₁ ou C₂ ; et
R₄ représente
(a) un groupe -CO-CHPh-O-(alkanoyle en C₁ à C₄)
(b) -CO-CH₂-S(O)₀₋₂-CH₃
(c) -CS-(alkyle en C₁ à C₄)
(d) -CO-CH₂-O-CO₂-(CH₂)₂-OCH₃
(e) -CO-CH₂-O-CO-NH(alkyle en C₁ à C₃)
(f) -CO-(CH₂)₀₋₁-CO-CH₃, ou
(g) -CN.

2. Composé suivant la revendication 1, qui a la configuration optique de la formule Ia

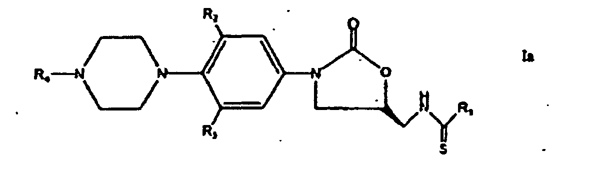

3. Composé suivant la revendication 1 ou la revendication 2, dans lequel R₁ représente un groupe alkyle en C₁ à C₄.

4. Composé suivant la revendication 3, dans lequel R₁ représente un groupe éthyle.

5. Composé suivant l'une quelconque des revendications précédentes, dans lequel R₂ et R₃ représentent indépendamment H ou F.

6. Composé suivant la revendication 5, dans lequel R₂ représente H et R₃ représente F.

7. Composé suivant la revendication 1, qui est :
le N {[(5S)-3-(3-fluoro-4-{4-[2-(méthylsulfinyle) acétyle]-1-pipérazinyle}phényle)-2-oxo-1,3-oxazolidine-5-yl]méthyle}propanethioamide.
le N-{[(5S)-3-(3-fluoro-4-{4-[2-(méthylsulfanyle) acétyle]-1-pipérazinyle}phényle)-2-oxo-1,3-oxazolidine-5-yl]méthyle}propanethioamide, ou
le N-{[(5S)-3-(3-fluoro-4-{4-[2-(méthylsulfonyle) acétyle]-1-pipérazinyle}phényle)-2-oxo-1,3-oxazolidine-5-yl]méthyle}propanethioamide

8. Composé suivant la revendication 1, qui est le N-({(5S)-3-[4-(4-cyano-1-pipérazinyle)-3-fluorophényle]-2-oxo-1,3-oxazolidine-5-yl}méthyle)propanethioamide.

9. Composé suivant la revendication 1, qui est
le N-({(5S)-3-(3-fluoro-4-{4-[2-(méthylamino-carbonyloxy)acétyle]-1-pipérazinyle}phényle)-2-oxo-1,3-oxazolidine-5-yl}méthyle)propanethioamide,
le N-({(5S)-3-(3-fluoro-4-{4-[2-[(2-méthoxyéthoxy) carbonyloxy]acétyle]-1- pipérazinyle}phényle)-2-oxo-1,3-oxazolidine-5-yl}méthyle)propanethioamide ou
le N-({(5S)-3-(3-fluoro-4-{4-acétoacétyle-1-pipérazinyle) phényle]-2-oxo-1,3-oxazolidine-5-yl}méthyle) propanethioamide.

10. Composé suivant la revendication 1, qui est le N-({(5S)-3-[3-fluoro-4-(4-pyruvoyle-1-pipérazinyle)phényle]-2-oxo-1,3-oxazolidine-5-yl}méthyle)propanethioamide.

11. Composé suivant la revendication 1 qui est le N-({(5S)-3-[4-(4-éthanethiolyle-1-pipérazinyle)-3-fluoro-phényle]-2-oxo-1,3-oxazolidine-5-yl}méthyl)propanethioamide.

12. Composé suivant la revendication 1 qui est le N-[((5S)-3-(3-fluoro-4-[4-((2S)-2-acétoxy-2-phénylacétyle)-1-pipérazinyl]phényle}-2-oxo-1,3-oxazolidine-5-yl)méthyle]-propanethioamide.

13. Composition pharmaceutique comprenant un composé suivant l'une quelconque des revendications précédentes et un support pharmaceutiquement acceptable.

14. Utilisation d'un composé suivant l'une quelconque des revendications 1 à 12 pour la production d'un médicament destiné au traitement d'une infection microbienne chez un mammifère.

15. Utilisation suivant la revendication 14, dans laquelle le médicament est destiné à être administré par voie orale, parentérale, transdermique ou topique.

16. Utilisation suivant la revendication 14 ou la revendication 15, dans laquelle le composé est destiné à être administré en une quantité de 0,1 à 100 mg/kg de poids corporel du mammifère/jour.

17. Utilisation suivant la revendication 16, dans laquelle la quantité est comprise dans l'intervalle de 1 à 50 mg/kg du poids corporel du mammifère/jour.

18. Utilisation suivant l'une quelconque des revendications 14 à 17, dans laquelle l'infection est une infection cutanée.

19. Utilisation suivant l'une quelconque des revendications 14 à 17, dans laquelle l'infection est une infection ophtalmique.
